# EUROPEAN PATENT APPLICATION

(11) **EP 4 578 542 A1**
(43) Date of publication of application: **02.07.2025**
(21) Application number: 24209511.5
(22) Date of filing: 29.10.2024
(51) Int. Cl.: B01J 19/00, G01N 21/62

(54) **SOLID SUBSTRATE AND BIOCHIP**

(30) Priority: 29.12.2023 CN 202311862163
(71) Applicant: GeneMind Biosciences Company Limited, Shenzhen City, 518023 Guangdong (CN)
(72) Inventor: WANG, Wenbing, Shenzhen City, 518023 (CN); LIN, Zhifeng, Shenzhen City, 518023 (CN); LI, linsen, Shenzhen City, 518023 (CN); CHEN, Fang, Shenzhen City, 518023 (CN)
(74) Representative: Michalski Hüttermann & Partner Patentanwälte mbB

(57) **Abstract**

The present disclosure relates to the technical field of bioassay devices, and in particular, to a solid substrate and a biochip thereof. A surface of the solid substrate disclosed herein is provided with a plurality of sets of regularly arranged marking units. Each set of marking units comprises at least three marks arranged according to a preset pattern. Each mark comprises a first trackline arranged in a first direction and a second trackline arranged in a second direction and intersected with the first trackline. The present disclosure significantly improves the recognizability of the tracklines by redesigning the marking units on the solid substrate, thereby improving the accuracy of calibration.

## Description

### TECHNICAL FIELD

The present disclosure relates to the field of gene assay devices, and in particular, to a solid substrate and a biochip thereof.

### BACKGROUND

Gene sequencing chips are an important consumable for gene sequencing and play an important role in gene sequencing. Specifically, the chip surface is processed to bind to chemical molecules or biomolecules. Nucleic acid molecules can bind to the chemical molecules or biomolecules by means of covalent bonds, hydrogen bonds, etc., and are thus immobilized on the chip surface. In the process of base recognition of nucleic acid molecules (also referred to as nucleic acid templates) immobilized on the chip surface based on fluorescent signals, the fluorescent signals of nucleotides or their analogs introduced into the nucleic acid molecules in each cycle of base extension can be captured by optical imaging, thereby acquiring the base types of the incorporated nucleotides or their analogs according to the fluorescent signals.

For ease and precision of identifying the nucleotide type introduced in each cycle of base extension, surface marks are required on the sequencing chip to locate the fluorescent signal in the images. How to reduce the influence on the performance of sequencing chips while effectively identifying the marks is a problem continuously concerning the industry.

### SUMMARY

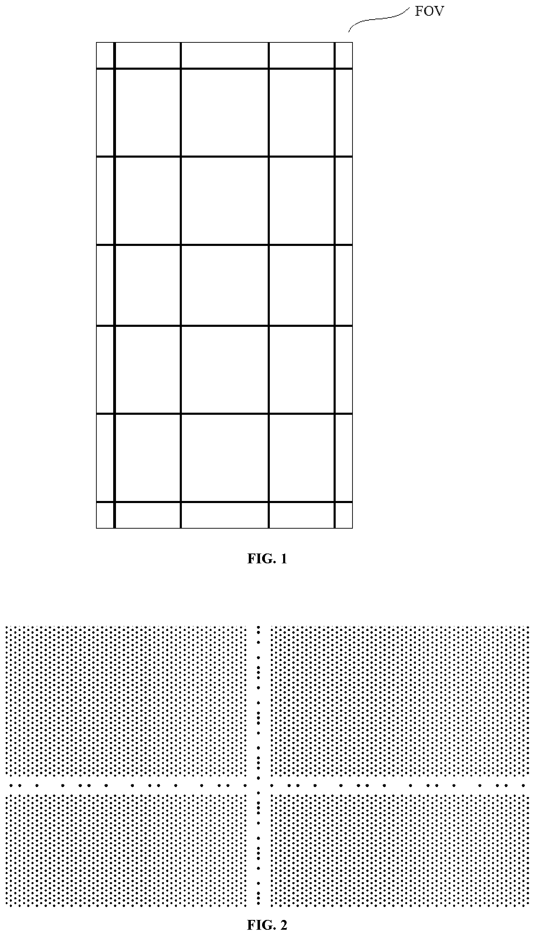

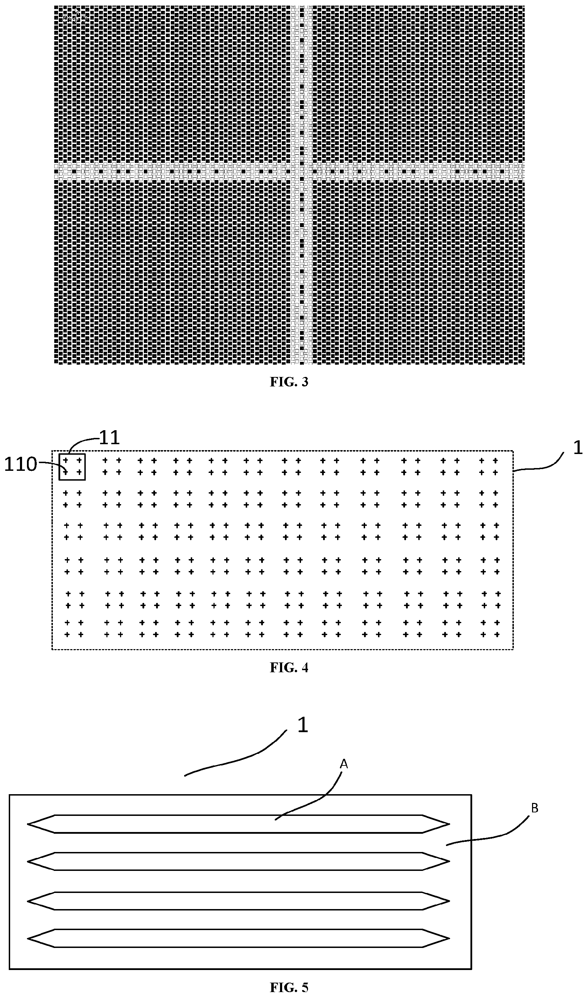

In the gene sequencing chip, tracklines are designed on the surface of chips to achieve the coarse positioning of a standard FOV and the fine positioning of a signal. Specifically, the standard FOV is positioned by predesigned tracklines (such as a preset pattern of tracklines), then the position of the nucleic acid molecule in the sequencing region is determined according to the signal position displayed by the tracklines, and finally, the base signals at the position of the nucleic acid molecule in the sequencing region are acquired. As an example, as shown in FIG. 1, the tracklines in a standard FOV consist of 4 × 6 intersecting (e.g., perpendicular) lines arranged at intervals according to a certain rule to divide the FOV into a plurality of regularly arranged blocks. Enlarged schematic views of the tracklines are shown in FIGs. 2 and 3, taking two intersecting lines as an example. As can be seen from the figures, in a FOV, the area defined by the tracklines has a width of 7 pixels in the transverse direction (hereinafter referred to as the X direction) and a length of 5 pixels in the longitudinal direction (hereinafter referred to as the Y direction) perpendicular to the transverse direction. The area defined by tracklines according to such a design accounts for a large portion of the FOV. Since the tracklines do not contribute to the sequencing throughput, they may cause a loss in the throughput of gene sequencing chips.

In order to solve the above problems, the present application provides a solid substrate and a biochip capable of accurately recognizing a trackline and beneficial to improving the throughput.

In a first aspect, the present application provides a solid substrate, where a surface of the solid substrate is provided with a plurality of sets of regularly arranged marking units; each set of marking units includes at least three marks arranged according to a preset pattern; each mark includes a first trackline arranged in a first direction and a second trackline arranged in a second direction and intersected with the first trackline. As one possible implementation of the solid substrate of the present application, the solid substrate is provided with at least one detection region, and each detection region is provided with at least one set of marking units.

As one possible implementation of the solid substrate of the present application, the detection region includes one or more detection units, and each detection unit is provided with one set of marking units. As one possible implementation of the solid substrate of the present application, the detection region includes a plurality of detection rows arranged in the first direction.

As one possible implementation of the solid substrate of the present application, the distances between adjacent detection rows are identical.

As one possible implementation of the solid substrate of the present application, a plurality of uniformly spaced detection sites are arranged in the same row.

As one possible implementation of the solid substrate of the present application, the detection sites are micron- or nano-pores.

As one possible implementation of the solid substrate of the present application, the detection sites are nano-scale or micron-scale protrusions.

As one possible implementation of the solid substrate of the present application, the detection sites in the detection region have the same size.

As one possible implementation of the solid substrate of the present application, in two adjacent rows, except for at least one of the two detection sites at the two ends of each row, each detection site on one row is located on the perpendicular bisector of the line segment between two adjacent detection sites on the other row.

As one possible implementation of the solid substrate of the present application, the detection unit includes a rectangular region block located in a central region of the detection unit.

As one possible implementation of the solid substrate of the present application, the marking unit is arranged in the rectangular region block.

As one possible implementation of the solid substrate of the present application, in one set of marking units, the area of the region defined by the connecting lines of the marks accounts for 50% or more of the total area of the rectangular region block.

As one possible implementation of the solid substrate of the present application, in one set of marking units, the area of the region defined by the connecting lines of the marks accounts for 80% or more of the total area of the rectangular region block.

As one possible implementation of the solid substrate of the present application, in one set of marking units, a pattern defined by the connecting lines of the marks is a symmetrical pattern.

As one possible implementation of the solid substrate of the present application, the center of the symmetric pattern coincides with the center of the detection unit.

As one possible implementation of the solid substrate of the present application, each set of marking units includes three marks arranged according to the preset pattern.

As one possible implementation of the solid substrate of the present application, the pattern defined by the connecting lines of the three marks is an isosceles triangle.

As one possible implementation of the solid substrate of the present application, each set of marking units includes four marks arranged according to the preset pattern.

As one possible implementation of the solid substrate of the present application, the pattern defined by the connecting lines of the four marks is a rectangle.

As one possible implementation of the solid substrate of the present application, the detection unit includes the rectangular region block located in the central region of the detection unit, and sides of the rectangular region block are parallel or perpendicular to the first direction.

As one possible implementation of the solid substrate of the present application, the four marks are respectively arranged at four vertices of the rectangular region block.

As one possible implementation of the solid substrate of the present application, the first direction and the second direction are perpendicular.

As one possible implementation of the solid substrate of the present application, the total area of the marking units 11 accounts for 1% or less of the surface area of the detection unit 10A.

As one possible implementation of the solid substrate of the present application, the first trackline has an aspect ratio of 15-30:1.

As one possible implementation of the solid substrate of the present application, the second trackline has an aspect ratio of 15-30: 1.

As one possible implementation of the solid substrate of the present application, the length ratio of the first trackline to the second trackline is 0.8-1.2: 1.

As one possible implementation of the solid substrate of the present application, the solid substrate includes a plurality of detection units, and each detection unit is provided with one set of marking units; the detection unit is provided with a plurality of uniformly spaced detection sites arranged in the first direction.

As one possible implementation of the solid substrate of the present application, according to the distance *l* between centers of two adjacent detection sites in the first direction, lengths and widths of the first trackline and the second trackline satisfy at least one of the following conditions:
the first trackline has a length of 80-150 *l*,
the second trackline has a length of 80-150 *l*,
the first trackline has a width of 3-8 *l* ,
the second trackline has a width of 3-8 *l* .

As one possible implementation of the solid substrate of the present application, the first trackline and/or the second trackline are grooves formed on the surface, and a recognizable mark is arranged in the groove.

As one possible implementation of the solid substrate of the present application, the solid substrate includes a plurality of detection units, and each detection unit is provided with one set of marking units; the detection unit is provided with a plurality of uniformly spaced detection sites arranged in the first direction; the distance between two adjacent recognizable marks in the groove is less than the distance between two adjacent detection sites in the detection unit.

As one possible implementation of the solid substrate of the present application, the distance between the recognizable marks in the groove is 1/2 or less of the distance between adjacent detection sites in the detection unit.

As one possible implementation of the solid substrate of the present application, the first trackline and/or the second trackline includes pores or protrusions formed on the surface of the solid substrate, and at least part of the pores or protrusions are provided with a recognizable mark.

As one possible implementation of the solid substrate of the present application, in the first trackline, the center-connecting lines of the pores or protrusions form a straight line, and the straight line is parallel to the first direction.

As one possible implementation of the solid substrate of the present application, in the second trackline, the center-connecting lines of the pores or protrusions form a straight line, and the straight line is perpendicular to the first direction.

As one possible implementation of the solid substrate of the present application, the distances between the adjacent recognizable marks on the first trackline and/or the second trackline are separately equal.

As one possible implementation of the solid substrate of the present application, the solid substrate includes a plurality of detection units, and each detection unit is provided with one set of marking units; the detection unit is provided with a plurality of uniformly spaced detection sites arranged in the first direction; the distance between at least part of adjacent recognizable marks is greater than the distance between two adjacent detection sites.

As one possible implementation of the solid substrate of the present application, according to the distance *l* between centers of two adjacent detection sites in the first direction, the distance between adjacent recognizable marks is n folds of *l*, and n is an integer greater than or equal to 1.

As one possible implementation of the solid substrate of the present application, n is a positive integer of 2-4.

As one possible implementation of the solid substrate of the present application, the recognizable marks are regularly distributed on the first trackline according to a first arrangement rule, and the recognizable marks are regularly distributed on the second trackline according to a second arrangement rule.

As one possible implementation of the solid substrate of the present application, the recognizable marks are arranged in all pores or on the surface of all protrusions of the first trackline and/or the second trackline. As one possible implementation of the solid substrate of the present application, the pores are at least one of circular pores, elliptical pores, polygonal pores, and irregular pores.

As one possible implementation of the solid substrate of the present application, the pores are at least one of circular pores and regular polygonal pores.

As one possible implementation of the solid substrate of the present application, the shapes and sizes of the pores arranged on the surface where the first trackline and/or the second trackline are located are identical. As one possible implementation of the solid substrate of the present application, the protrusions are at least one of circular protrusions, elliptical protrusions, polygonal protrusions, and irregular protrusions.

As one possible implementation of the solid substrate of the present application, the protrusions are at least one of circular protrusions and regular polygonal protrusions.

As one possible implementation of the solid substrate of the present application, the shapes and sizes of the protrusions arranged on the surface where the first trackline and/or the second trackline are located are identical.

As one possible implementation of the solid substrate of the present application, the recognizable mark is a fluorescent mark.

In a second aspect, the present application provides a biochip, including the solid substrate according to the first aspect.

The present disclosure, by designing a plurality of sets of regularly arranged marking units on the solid substrate, can achieve the coarse positioning of surface regions on the solid substrate and the fine positioning of surface positions on the solid substrate based on the arrangement rule of the marking units and the relation between the first tracklines and the second tracklines. Moreover, the positioning on the surface of the solid substrate by means of the arrangement of intersecting marking units can reduce the occupied area of the marks on the surface of the solid substrate and the area loss on the surface of the solid substrate and thus improve the valid utilization rate of the surface of the solid substrate. When the solid substrate is used as a biochip, the sequencing throughput can be improved.

### BRIEF DESCRIPTION OF THE DRAWINGS

In order to more clearly illustrate the technical solutions in the examples of the present application, the drawings required for use in the description of the examples will be briefly described below. It is obvious that the drawings in the description below are only examples of the present application, and other drawings can be derived from the provided drawings by those of ordinary skill in the art without making creative efforts.
FIG. 1 illustrates a schematic view of a FOV with tracklines according to the prior art;
FIG. 2 illustrates an enlarged schematic view of two intersecting tracklines in FIG. 1;
FIG. 3 illustrates an enlarged schematic view of two intersecting tracklines in FIG. 1;
FIG. 4 illustrates a schematic view of the solid substrate with the marking units according to the examples of the present application;
FIG. 5 illustrates another structural schematic view of the solid substrate according to the examples of the present application;
FIG. 6 illustrates a partial schematic view of the detection unit according to the examples of the present application;
FIG. 7 illustrates a schematic view of the arrangement of detection sites according to the examples of the present application;
FIG. 8 illustrates a schematic view of the rectangular region block in the detection unit according to the examples of the present application;
FIG. 9 illustrates a schematic view of the effect of providing continuous recognizable signals in a groove according to the examples of the present application;
FIG. 10 illustrates a schematic view of the marking unit including three marks according to the examples of the present application;
FIG. 11 illustrates a schematic view of the marking unit including four marks according to the examples of the present application;
FIG. 12 illustrates a schematic view of the detection unit including the marking units according to the examples of the present application;
FIG. 13 illustrates a partially schematic view of a mark according to the examples of the present application;
FIG. 14 illustrates a partially schematic view of another mark according to the examples of the present application;
FIG. 15 illustrates a schematic view of the first repeating unit according to the examples of the present application;
FIG. 16 illustrates a schematic view of the second repeating unit according to the examples of the present application;
FIG. 17 illustrates a structural schematic view of the comparative example of the present application;
FIG. 18 illustrates Q30 curves for Example 1 (V03) and Comparative Example 1 (V02);
FIG. 19 illustrates error rate curves for Example 1 (V03) and Comparative Example 1 (V02);
FIG. 20 illustrates Q30 curves for Example 2 (V04) and Comparative Example 1 (V02); and
FIG. 21 illustrates error rate curves for Example 2 (V04) and Comparative Example 1 (V02).

Description of reference numerals:
1, A solid substrate; 10A, detection unit; 10A1, rectangular region block; 11, marking unit; 110, mark; 1101, first trackline; 1102, second trackline; A, detection region; A1 (A11/A12), detection row; A10, detection site; 110 and A11/A12/A13/A14/A15/A16/A17/A19, detection site; O1/O2/O3/O4, intersection; 101, detection region; 102, first trackline; 103, second trackline; 105, detection site (comparative example).

### DETAILED DESCRIPTION

The present disclosure will be illustrated in further detail with reference to the following detailed description and drawings. In the following embodiments, numerous specific details are given to provide a thorough understanding of the present application. However, those skilled in the art will readily recognize that some of the features may be omitted or substituted with other features and methods in different instances. In some instances, certain operations related to the present application are not illustrated or described in this specification to avoid obscuring the key part of the present application with unnecessary detail. For those skilled in the art, it is not necessary to describe in detail these related operations, as such operations can be fully understood from the description in the specification and the general knowledge in the art.

Furthermore, the illustrated features, operations, or characteristics may be combined in any suitable manner to give various embodiments. Also, the various procedures or actions in the description of the methods may be exchanged or adjusted in order, as will be apparent to those skilled in the art. Thus, the various orders in the specification and drawings are for the purpose of clearly illustrating certain embodiments only and are not intended to imply a necessary order unless otherwise indicated that a certain order is necessary.

The serial numbers used herein for the components, such as "first", "second", etc., are used merely to distinguish between the objects described, and do not carry any sequential or technical meaning.

In the embodiments of the present application, the term "sequencing" may also be referred to as "nucleic acid sequencing" or "gene sequencing". The three are used interchangeably and refer to the determination of the type and order of bases or nucleotides (including nucleotide analogs) in a nucleic acid molecule. The sequencing involves the process of binding nucleotides to a template and acquiring the corresponding signals emitted by the nucleotides (including analogs). The so-called sequencing includes sequencing by synthesis (SBS) and/or sequencing by ligation (SBL), DNA sequencing and/or RNA sequencing, and long fragment sequencing and/or short fragment sequencing (the long fragment and short fragment are defined relatively; e.g., nucleic acid molecules longer than 1 kilobase pair (kb), 2 kb, 5 kb, or 10 kb may be referred to as long fragments, and nucleic acid molecules shorter than 1 kb or 800 bp may be referred to as short fragments).

The sequencing generally involves cycles of process to achieve the determination of the type and order of multiple bases or nucleotides on a nucleic acid template. The examples of the present application refer to each cycle of the "process to achieve the determination of the type and order of multiple bases or nucleotides on a nucleic acid template" as a "cycle of sequencing". The "cycle of sequencing", also referred to as "sequencing cycle", may be defined as the completion of one base extension of four types of nucleotides/bases; in other words, the "cycle of sequencing" may be defined as the determination of the base or nucleotide type at any given position on the template. For sequencing platforms that achieve sequencing based on polymerization or ligation reactions, the cycle of sequencing includes the process of binding four types of nucleotides (including nucleotide analogs) to the nucleic acid template at a time according to the base complementary rule and acquiring the corresponding signals emitted. For platforms that achieve sequencing based on polymerization reaction, a reaction system includes reaction substrate nucleotide, polymerase, and a nucleic acid template. A sequence fragment (a sequencing primer) binds to the nucleic acid template, and on the basis of the base pairing rule and the principle of the polymerization reaction, the added reaction substrate nucleotides are connected to the sequencing primer under the catalysis of the polymerase to realize the binding of a nucleotide to a specific position on the nucleic acid template. Generally, the cycle of sequencing may include one or more base extensions (repeats). For example, the four types of nucleotides are sequentially added to the reaction system, and the system is separately subjected to base extension and the acquisition of corresponding reaction signals, where each cycle of sequencing includes four base extensions; for another example, the four types of nucleotides are added into the reaction system in any combinations (such as in pairs or in one-three combinations), and the system is separately subjected to base extension and the acquisition of corresponding reaction signals as per the two combinations, where each cycle of sequencing includes two base extensions; for yet another example, the four types of nucleotides are added simultaneously to the reaction system for base extension and reaction signal acquisition, where the cycle of sequencing includes one base extension.

As used herein, the "trackline" is also referred to as a calibration line, recognition line, or trajectory line, and mainly serves to mark the standard FOV and locate the signal position, such that the correspondence between the detection position and the detection signal can be confirmed according to the trackline during the detection process.

As used herein, the term "sequencing" may also be referred to as "nucleic acid sequencing" or "gene sequencing". The three are used interchangeably and refer to the determination of the type and order of bases in a nucleic acid sequence.

As used herein, the "FOV" refers to the field of view. The application of the solid substrate includes a process of performing cycles of optical imaging (photographing) on a fixed region of the solid substrate by using an optical camera, and the region photographed by the optical camera each time is referred to as a FOV (field of view).

As used herein, the "read" refers to the sequencing result and/or the sequencing data obtained by the sequencing reaction, i.e., the fragments read by the sequencing, and the length of the read is referred to as the read length.

When the solid substrate is optically imaged, the surface of the solid substrate is divided into a plurality of FOVs in consideration of the configuration of different optical systems, particularly, according to the shooting area of the optical camera. In the process of optically imaging the solid substrate, in order to determine the standard "FOV" and further position the detection signal of the detection site in the standard "FOV" to recognize the optical information corresponding to the fixed region on the solid substrate, the arrangement of marks or tracklines are required on the surface of the solid substrate. It can be seen that the design of the marks or tracklines relates to the positioning and recognition of signals in the detection region. Taking biochips as an example, when a biochip is optically imaged, optical images corresponding to a variety of FOVs are obtained since only one FOV can be imaged per imaging. Such different optical images can be positioned by the marks or tracklines on the surface of the biochip. In an existing marking mode, tracklines are designed on the surface of chips to achieve the coarse positioning of a standard FOV and the fine positioning of a signal. The tracklines generally consist of m × n lines perpendicular to each other, and each line has a certain width. For example, in some biochips, the width of the m lines in the transverse direction (hereinafter referred to as the X direction) is A pixels; the width of the n lines in the vertical direction (hereinafter referred to as the Y direction) is B pixels. If the length of each FOV in the X direction is L₁ and the length in the Y direction is L₂, the area occupied by the tracklines in each FOV is A pixels × L₁ × m + B pixels × L₂ × n. Illustratively, for a FOV having a length of 200 pixels in the X direction and a length of 200 pixels in the Y direction, when the tracklines consist of 4 × 6 lines perpendicular to each other (i.e., 4 lines are arranged in the X direction, and 6 lines in the Y direction), and the width of the m lines in the X direction is 7 pixels and the width of the n lines in the Y direction is 5 pixels, the area occupied by the trackline is 7 pixels × 200 pixels × 4 + 5 pixels × 200 pixels × 6. As such, in the FOV, the tracklines occupy a large area, which does not produce sequencing data and thus results in a loss of throughput for the biochips.

In view of this, the examples of the present application provide a marking method instead of tracklines for fine positioning through an algorithm. Furthermore, the area occupied by the marks provided by the examples of the present application is greatly reduced, and compared with tracklines, the present application has the advantage of increased throughput of the biochip.

Taking a sequencing biochip as an example, the design of marks on the surface of the solid substrate is described below. In this example, in order to better illustrate the concept of the present disclosure, a biochip having micropores arranged in an array is described as a representative. Specifically, a large number of micropores arranged in an array are formed on the surface of the biochip by boring, and biomolecules are immobilized in the micropores. For example, in a sequencing method using nucleic acid molecules as the biomolecules and optical signals as the detection signals, in each cycle of base extension reaction, a nucleic acid template in at least one micropore binds to a nucleotide or a nucleotide analog containing an optical label according to the base complementary rule, and an optical signal is correspondingly produced at the position of the bound nucleotide or nucleotide analog in the micropore, and is represented by a bright spot in an optical image. By providing specific marks on the surface of the sequencing chip different from those in the detection region, the positioning of the micropore array can be achieved. It will be appreciated that, in the examples of the present application, the sites are not necessarily present in the form of micropores, but every preset position for binding to the target samples under test theoretically capable of producing a detectable signal after a certain reaction can be regarded as a site. That is, in other examples, sites can be formed in other manners instead of boring that arranges the samples under test on the solid substrate in a desired arrangement and acquires detectable signals (e.g., optical signals) at the positions of the samples under test.

After the nucleic acid template is immobilized in the pores and nucleotides or nucleotide analogs having an optical label bind to the nucleic acid template according to the base complementary rule, optical signals are produced by the optical labels in the reaction sites and are represented by bright spots on an optical image. Non-pore regions are represented by dark spots on the optical image since no nucleic acid templates are present and thus no optical signals can be detected during the sequencing. By forming bands different from the sequencing region, a recognition mark can be formed, which in turn helps calibrate each FOV on the sequencing chip and position the sequencing positions in the FOV.

**In a first aspect**, in one example, a solid substrate is provided, where an image acquisition sensor images one FOV at a time and acquires an optical image of the FOV when acquiring signals on the solid substrate. The optical images of multiple FOVs consist of the overall optical image of the solid substrate. By reading the information (such as fluorescence intensity, etc.) in the optical image of the corresponding region and performing algorithmic analysis on the optical image, the detection results of the region are acquired. For example, the types of nucleotides or nucleotide analogs bound to the nucleic acid template in the base extension reactions are acquired.

As shown in FIGs. 4-12, the surface of the solid substrate 1 is provided with a plurality of sets of regularly arranged marking units 11 as optical signal positioning markings for detecting a sample under test on the surface of the solid substrate 1 on the basis of optical signals. FIG. 4 illustrates a partial region of the surface of the solid substrate 1 provided with the marking units 11.

According to the examples of the present application, for the convenience of illustrating the arrangement of the marking units 11, the surface of the solid substrate 1 provided with the marking units 11 can be divided into a plurality of substrate units 10. The substrate units 10 are part of the surface, that is, the surface of the solid substrate 1 provided with the marking units 11 consists of a plurality of substrate units 10. The substrate units 10 are not specifically shown on the surface of the solid substrate 1, and the marking units 11 are arranged in at least part of the substrate units 10.

According to the examples of the present application, the solid substrate 1 is provided with at least one detection region A, and each detection region A is provided with at least one set of marking units 11. It will be appreciated that the detection region according to the examples of the present application is a region for carrying the sample under test. When the solid substrate 1 is used as the substrate for detection, the sample under test may be carried by the whole of the detection region A or may be carried by part of the detection region A. In one possible embodiment, the surface of the solid substrate 1 where the marking units 11 are located is the detection region A, that is, one surface of the whole solid substrate 11 is used as the region for carrying the sample under test. In another possible embodiment, as shown in FIG. 5, the solid substrate 1 includes a plurality of detection regions A and a non-detection region B outside the detection regions A, and the plurality of detection regions A are spaced apart by the non-detection region B. It will be appreciated that the non-detection region, as opposed to the detection region, in the examples of the present application, refers to a region that does not carry a sample under test or serve as a signal detection region during signal detection.

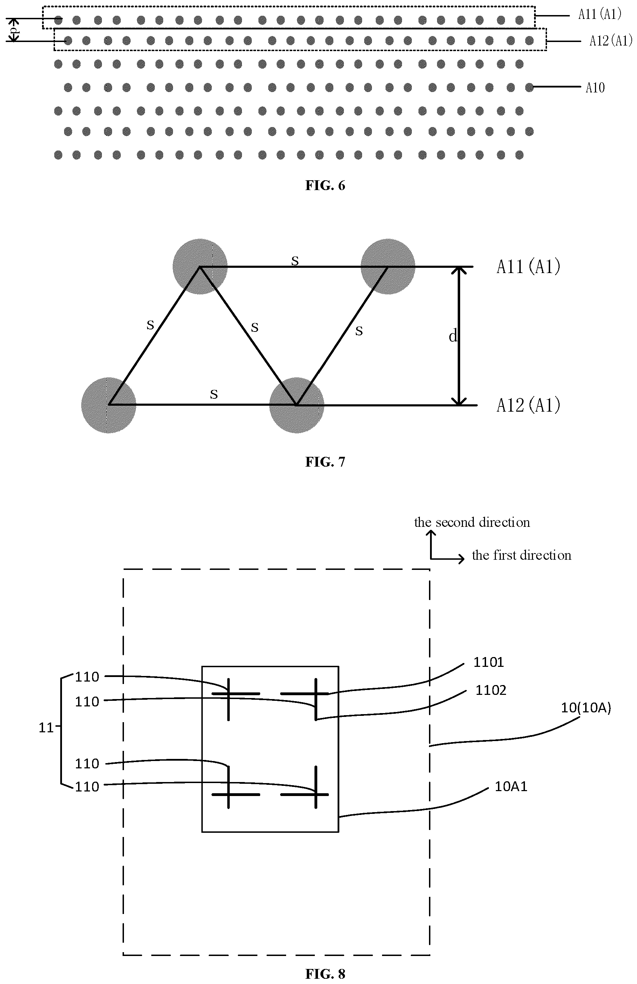

According to the examples of the present application, referring to FIG. 6, the detection region A includes a plurality of detection rows A1 arranged in the first direction, and each detection row A1 consists of a plurality of regularly arranged detection sites A10. In one possible implementation, among the plurality of detection sites A10 forming the detection row A1, adjacent detection sites A10 are uniformly spaced, that is, the plurality of uniformly spaced detection sites A10 are arranged in the same row A1. In the examples of the present application, when the detection sites A10 are in a regular shape selected from a circle, an ellipse, a rectangle, a square, a triangle, and other polygons, the distance between adjacent detection sites A10 is the length of the connecting line segment between the centers of adjacent detection sites A10; when the detection sites A10 are in an irregular shape, the center of the longest connecting line segment between any two points on the irregular circumference is taken as the center of the irregular pattern, and at this time, the distance between adjacent detection sites A10 is the length of the connection line segment between the centers of adj acent detection sites A10. It will be appreciated that at least the detection sites A10 in the same detection region A are shaped identically to facilitate positioning the signal sites in the detection region A. According to the examples of the present application, the detection sites A10 may have a variety of possible shapes. In one possible implementation, the detection sites A10 are configured as pore structures, that is, the detection sites 10 are formed by depression on the surface of the solid substrate 1. Illustratively, the detection sites A10 are micron- or nano-pores, and during the detection, the sample under test is immobilized in the micron-pores or nano-pores; in another possible implementation, the detection sites A10 are provided as protrusion structures, that is, the detection sites 10 are formed by protrusion from the surface of the solid substrate 1. Illustratively, the detection sites A10 are nano-scale or micron-scale protrusions. In still another possible implementation, the detection sites A10 are a pattern arranged on the surface of the solid substrate 1, that is, by performing surface processing on part of the solid substrate 1, the part of the surface of the solid substrate 1 can bind to the sample, while the remaining part cannot, thereby achieving the definition of the positions of the detection sites A10. It will be appreciated that the part of the surface of the solid substrate 1 includes a plurality of discrete regions (detection sites A10) that are dispersed between "the remaining part" that cannot bind to the sample.

According to the examples of the present application, in order to better facilitate the positioning of the signals produced by the samples under test bound on the surface of the solid substrate 1, the detection sites A10 in the detection region A have the same size.

According to the examples of the present application, the distances d between adjacent detection rows A1 (such as A11 and A12 in FIG. 6) are identical. In one possible implementation manner, as shown in FIG. 7, in two adjacent detection rows A1 (e.g., A11 and A12 in FIGs. 3 and 4), except for at least one of two detection sites A10 located at the two ends of the detection rows A1 (e.g., A11 and A12 in FIGs. 6 and 7), each detection site A10 on one detection row A11 is located on the perpendicular bisector of the line segment between two adjacent detection sites A10 on the other detection row A12. That is, the detection sites A10 form an equilateral triangle or parallelogram array between detection regions A. By providing such an array structure, it is advantageous to position and recognize the signals originating from these arrayed detection sites A10 by a construction algorithm.

According to the examples of the present application, the distribution of the marking units 11 on the surface of the solid carrier 1 can be set according to the size of the FOV when the surface of the solid substrate 1 is imaged. In one possible embodiment, the size of the substrate unit 10 corresponds to the size of the predetermined FOV for imaging the surface of the solid substrate 1. In some examples, the substrate unit 10 is a rectangular unit, a circular unit, or an oval unit.

In one possible embodiment, for the detection region A of the solid substrate 1, each substrate unit 10 is provided with a set of marking units 11. For ease of understanding, the substrate unit 10 in the detection region A is defined as the detection unit 10A (a size corresponding to the size of one predetermined FOV for imaging the surface), as shown in FIG. 8. That is, the detection region A includes one or more detection units 10A, and each detection unit 10A is provided with one set of marking units 11. By means of the set of marking units 11, the positioning of the optical signals in the surface region corresponding to one FOV, i.e., the detection unit 10A, is achieved.

According to the examples of the present application, the detection unit 10A includes the rectangular region block 10A1 located in the central region of the detection unit 10A, and the rectangular region block 10A1 is formed by extending from the center of the detection unit 10A radially to the periphery. In one possible implementation, the rectangular region block 10A1 is a region formed by at least 50% retraction of the detection unit 10A under the precondition that the center of the detection unit 10A is not changed. In one possible embodiment, the detection unit 10A is a rectangle, and the rectangular region block 10A1 is a region formed by at least 50% retraction of the two pairs of opposite sides of the rectangular detection unit 10A along the axes of symmetry. It will be appreciated that the two pairs of opposing sides of rectangular detection unit 10A may be retracted by the same proportion, such as 50% each; alternatively, the two pairs of opposing sides of rectangular detection unit 10A may be retracted by different proportions, for example, one pair of opposite sides of rectangular detection unit 10A are retracted by 50% along the central axis of symmetry and the other pair of opposite sides of rectangular detection unit 10A are retracted 60% along the central axis of symmetry. When the two pairs of opposite sides of the rectangular detection unit 10A are retracted by unequal proportions, the rectangular region block 10A1 is the region defined by the extended lines of the two pairs of retracted opposite sides.

According to the examples of the present application, the marking units 11 are arranged in the rectangular region block 10A1. As such, by arranging the marking units 11 in the 50% range of the center region of the detection unit 10A and the design features of the marking units 11, the detection of every signal produced in the detection unit 10A can be achieved. In some examples, in one set of marking units 11, the area of the region defined by the connecting lines of the marks 110 accounts for 50% or more of the total area of the rectangular region block 10A1; illustratively, the area of the region defined by the connecting lines of the marks 110 accounts for 50%, 55%, 60%, 65%, 70%, 75%, 80%, 85%, 90%, 95%, or 100% of the total area of the rectangular region block 10A1. In one specific example, in one set of marking units 11, the area of the region defined by the connecting lines of the marks 110 accounts for 80% or more of the total area of the rectangular region block 10A1.

According to the examples of the present application, each set of marking units 11 includes at least three marks 110 arranged according to the preset pattern. As such, the positioning and attribution of the signals within one FOV can be achieved by the arrangement and distribution of three or more marks 110 in the detection unit 10A. In the examples of the present application, for a set of marking units 11, the number of the marks 110 included therein is at least three, and may be 3, 4, 5, 6, 7, 8, or even more. However, it will be appreciated that, for marks 110 with the same size, a greater number may be more favorable for the positioning of the signals in the detection unit 10A, but may also result in a greater occupied area and a smaller valid area. As such, in one possible embodiment, each set of marking units 11 includes 3-6 markings 110 arranged according to a preset pattern.

In some possible embodiments, in one set of marking units 11, a pattern defined by the connecting lines of the marks 110 is a symmetrical pattern, for example, an isosceles triangle (especially an equilateral triangle), a rectangle such as a rectangle or a square, or other symmetrical polygons such as a diamond, a regular pentagon, a regular hexagon, etc., and theoretically, other patterns such as a circle, an ellipse, etc. As such, by configuring the pattern defined by the connection lines of the marks 110 as a symmetrical pattern, the positioning of the signals inside and outside the symmetrical pattern can be achieved by means of an algorithm. It will be appreciated that the pattern defined by the connecting lines of the marks 110 may be a pattern defined by sequentially connecting the centers of the marks 110. In some examples, the center of the symmetric pattern coincides with the center of the detection unit 10A.

In one specific example, each set of marking units 11 includes three markings 110 arranged according to a preset pattern. In some examples, the pattern defined by the connecting lines of the three marks 110 is an isosceles triangle. In some examples, the area of the isosceles triangle accounts for 20% or more of the total area of the detection unit 10A; illustratively, the area of the isosceles triangle accounts for 20%, 25%, 30%, 35%, 40%, 45%, 50%, etc., of the total area of the detection unit 10A.

In another specific example, each set of marking units 11 includes 4 markings 110 arranged according to a preset pattern. In some examples, the pattern defined by the connecting lines of the four marks 110 is a rectangle. In some examples, the area of the rectangle accounts for 20% or more of the total area of the detection unit 10A; illustratively, the area of the rectangle accounts for 20%, 25%, 30%, 35%, 40%, 45%, 50%, etc., of the total area of the detection unit 10A.

According to the examples of the present application, as shown in FIG. 8, each mark 110 includes a first trackline 1101 arranged in a first direction and a second trackline 1102 arranged in a second direction and intersected with the first trackline. In the examples of the present application, the intersection of the first trackline 1101 and the second trackline 1102 is defined as the center of the mark 110. In one example where each set of marking units 11 includes 4 markings 110 arranged according to a preset pattern and the pattern defined by the connecting lines of the four marks 110 is a rectangle, the sides of the rectangle are parallel or perpendicular to the first direction.

In one possible embodiment, in the marking unit 11, at least one of the first trackline 1101 and the second trackline 1102 of each mark 110 coincides with four sides of the rectangular region block 10A1 or extension lines thereof. In some examples, the detection unit 10A includes the rectangular region block 10A1 located in the central region of the detection unit 10A, and sides of the rectangular region block 10A1 are parallel or perpendicular to the first direction. Illustratively, when the marking unit 11 consists of three marks 110, the pattern defined by the connecting lines of the three marks 110 is an isosceles triangle, and the first tracklines 1101 of the three marks 110 are arranged on a pair of opposite sides of the rectangular region block 10A1. When the marking unit 11 consists of four marks 110, the pattern defined by the connecting lines of the four marks 110 is a rectangle, and the first tracklines 1101 of the four marks 110 are arranged on a pair of opposite sides of the rectangular region block 10A1.

According to the examples of the present application, the first tracklines 1101 and the second tracklines 1102 intersect at a preset angle, and through the preset angle and the positions of the first tracklines 1101 and the second tracklines 1102 in the detection unit 10A, the attribution of each signal positioned in the detection unit 10A can be calculated by means of an algorithm.

According to the examples of the present application, the included angle between the first trackline 1101 and the second trackline 1102 may be set according to the number of marks 11 in the detection unit 10A, and by setting the included angle formed by the first trackline 1101 and the second trackline 1102 and the detection row A1, the attribution of each signal in the detection unit 10A can be determined by these parameters.

According to the examples of the present application, the included angle between the first trackline 1101 and the detection row A1 may be 0-180°. In some examples, the first trackline 1101 is parallel to detection row A1. In one example, the first direction and the second direction are perpendicular. That is, the first tracklines 1101 and the second tracklines 1102 are arranged perpendicular.

In one possible implementation, referring to FIG. 8, the detection unit 10A includes one set of marking units 11, and the marking units 11 include four marks 110. The mark 110 includes a first trackline 1101 and a second trackline 1102 perpendicular to each other, where the first trackline 1101 is parallel or perpendicular to the detection row A1 (not shown).

According to the examples of the present application, the arrangement of the marks 110 in the detection unit 10A can be flexible on the basis of achieving the marking. As one possible implementation of the present application, the detection sites A10 in the detection region A is a groove or a protrusion formed on the surface of the solid substrate 1, or a pattern formed by physical and/or chemical processing on the surface of the solid substrate 1.

As one possible implementation, the first trackline 1101 and/or the second trackline 1102 are grooves formed on the surface of the solid substrate 1, specifically, grooves in the detection unit 10A. As such, the recognition and positioning of signals within the detection unit 10A can be achieved by generating detection signals within the grooves that are different from those of the surrounding detection sites A10.

In one possible embodiment, recognizable marks are arranged within the groove. The detection sites A10 are capable of binding to the recognizable marks or are capable of binding to the recognizable marks via the immobilized sample, and the recognizable marks are arranged within the groove in a manner different from that in the detection sites A10 outside the groove. As such, the recognizable marking units 11 are recognized by means of the difference in the recognizable signals produced by the recognizable marks, and the positioning results of the detectable signals in all the detection sites A10 in the detection unit 10A are acquired by means of the positions and arrangement of the marks 110 of the detection unit 10A in the marking units 11. In the examples of the present application, the recognizable mark is a mark that can be recognized when a sample on the solid substrate 1 is detected, which includes, but is not limited to, a fluorescent mark.

In one specific embodiment, each detection region A includes a plurality of detection units 10A, and each detection unit 10A is provided with one set of marking units 11; the detection unit 10A is provided with a plurality of detection rows A10 in the first direction, and each detection row A1 is provided with a plurality of uniformly spaced detection sites A10. The marking unit 11 includes at least three marks 110, each mark 110 includes a first trackline 1101 arranged in the first direction, and a second trackline 1102 arranged in the second direction and intersecting the first trackline 1101. The first trackline 1101 and the second trackline 1102 are grooves formed in the detection unit 10A, and the recognizable marks are provided in the grooves. The distance between adjacent recognizable marks in the groove is less than the distance between adjacent detection sites in the detection unit. As such, the first trackline 1101 and the second trackline 1102 produce recognizable signals with a higher intensity than those of the surrounding regions, so as to achieve the positioning of the marking unit 11. The signals produced in each detection site 10A in the detection unit 10A are positioned according to the position of each mark 110 in the marking unit 11 and the arrangement features of the first trackline 1101 and the second trackline 1102 in each mark 110.

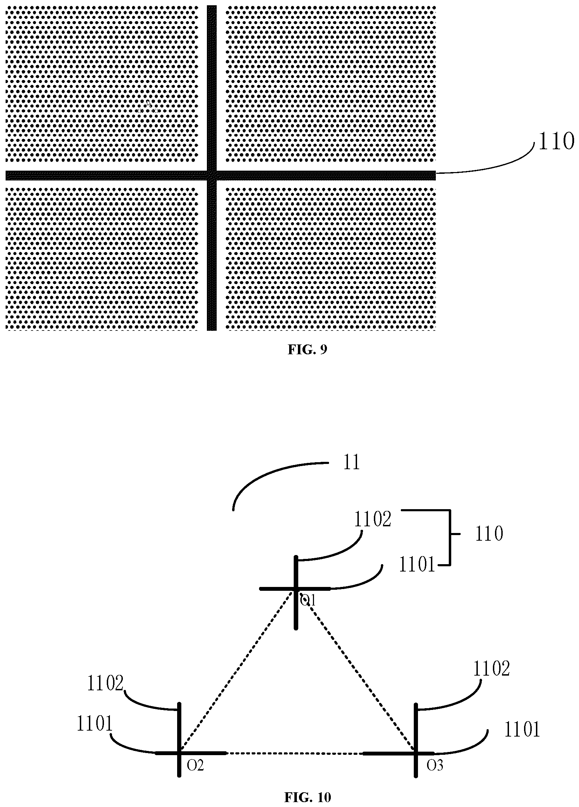

In some examples, the distance between the recognizable marks in the groove is 1/2 or less of the distance between adjacent detection sites 10A in the detection unit 10A. As such, the first trackline 1101 and the second trackline 1102 produce signals with a significantly higher intensity than those of the detection site 10A, such that the recognition of information such as the number, positions, orientation, and sizes of the marks 110 in the detection unit 10A is achieved by the difference in intensity, and the positioning of the signals in each detection site 10A in the detection unit 10A can be achieved by the information. In some examples, the density of the recognizable marks in the groove is 4 folds or more the density of the detection sites 10A in the detection unit 10A, such that a dense, intense signal band is produced in the whole groove to distinguish the signals produced by the detection sites 10A in the detection unit 10A and to accelerate the recognition of the first trackline 1101 and the second trackline 1102. Illustratively, as shown in FIG. 9, the marks 110 produce a continuous recognizable signal.

In another possible embodiment, no recognizable marks are arranged within the groove. The detection sites A10 are capable of binding to the recognizable marks or are capable of binding to the recognizable marks via the immobilized sample. As such, when the signals in the detection unit 10A are acquired, no recognizable marks are present in the groove, that is, no recognizable signals are produced. During the image acquisition, the marking unit 11 can be recognized by means of the signal difference, and further, by means of the positions and arrangement of the marks 110 in the marking unit 11, the detectable signals in the detection site A10 in the detection unit 10A can be acquired, and the positioning of the detectable signals can be achieved.

As one possible implementation of the present application, the first trackline 1101 and/or the second trackline 1102 includes pores or protrusions formed on the surface of the solid substrate 1, and at least part of the pores or protrusions are provided with a recognizable mark. As such, the recognition of the marks 110 in the detection unit 10A can be achieved by forming recognizable marks with a different density and arrangement from the peripheral detection sites A10 via the first tracklines 1101 and/or the second tracklines 1102, and further, the recognition and positioning of signals in the detection unit 10A can be achieved according to the positions of the marks 110 in the marking unit 11 and the arrangement features of the first tracklines 1101 and the second tracklines 1102 in the marks 110. In one embodiment, the recognizable marks are arranged in all pores or on the surface of all protrusions of the first trackline 1101 and/or the second trackline 1102.

In one possible embodiment, in the first trackline 1101, the center-connecting lines of the pores or protrusions form a straight line, i.e., the centers of the pores or protrusions in the first trackline 1101 are in the same straight line, and the straight line is parallel to the first direction.

In one possible embodiment, in the second trackline 1102, the center-connecting lines of the pores or protrusions form a straight line, i.e., the centers of the pores or protrusions in the second trackline 1102 are in the same straight line, and the straight line is perpendicular to the first direction.

In some examples, the distances between adjacent recognizable marks on the first trackline 1101 are equal, i.e., the first trackline 1101 is provided with a plurality of uniformly spaced recognizable marks, and the distance between adjacent recognizable marks is different from the distance between adjacent detection sites 10A. In some examples, the distances between adjacent recognizable marks on the second trackline 1102 are equal, i.e., the second trackline 1102 is provided with a plurality of uniformly spaced recognizable marks, and the distance between adjacent recognizable marks is different from the distance between adjacent detection sites 10A. In some examples, the distances between adjacent recognizable marks on the first trackline 1101 and/or the second trackline 1102 are separately equal.

In some other examples, not all of the distances between adjacent recognizable marks on the first trackline 1101 are equal. In some examples, not all of the distances between adjacent recognizable marks on the second trackline 1102 are equal.

In one implementation, the solid substrate 1 includes a plurality of detection units 10A, and each detection unit 10A is provided with one set of marking units 11. The marking unit 11 includes at least three marks 110, each mark 110 includes a first trackline 1101 arranged in the first direction, and a second trackline 1102 arranged in the second direction and intersecting the first trackline 1101. The first trackline 1101 and the second trackline 1102 include pores or protrusions formed on the surface of the solid substrate 1, and the pores or protrusions are provided with a recognizable mark. The detection unit 10A is provided with a plurality of uniformly spaced detection sites A10 in the first direction, and at least part of the distances between adjacent recognizable marks on the first trackline 1101 and the second trackline 1102 are greater than the distance between two adjacent detecting sites A10. Illustratively, all of the distances between adjacent recognizable marks on the first trackline 1101 and the second trackline 1102 are greater than the distance between two adjacent detection sites A10. It will be appreciated that, in the examples of the present application, the distance between adjacent detection sites A10 refers to the length of the connecting line between the centers of two adjacent detection sites A10, and the centers are defined as above and are not recited here.

In some examples, according to the distance *l* between centers of two adjacent detection sites A10 in the first direction, the distance between adjacent recognizable marks on the first trackline 1101 and the second trackline 1102 is n folds of *l*, and n is an integer greater than or equal to 1. As such, the distribution of the signals produced by the recognizable marks on the first trackline 1101 and the second trackline 1102 is significantly different from that in the detection site A10, so as to achieve the positioning of the marking unit 11. The signals produced in each detection site 10A in the detection unit 10A are positioned according to the position of each mark 110 in the marking unit 11 and the arrangement features of the first trackline 1101 and the second trackline 1102 in each mark 110. In some examples, n is a positive integer of 2-4. Illustratively, n is 2, 3, or 4.

In the implementation that the first trackline 1101 and the second trackline 1102 include pores formed on the surface of the solid substrate 1, the pores are at least one of circular pores, elliptical pores, polygonal pores, and irregular pores. In some examples, the pores are at least one of circular pores and regular polygonal pores. In order to facilitate the recognition of the signals of the marks 110, preferably, the shapes and sizes of the pores arranged on the surface where the first trackline 1101 and the second trackline 1102 are located are identical.

In the implementation that the first trackline 1101 and the second trackline 1102 include protrusions formed on the surface of the solid substrate 1, the protrusions are at least one of circular protrusions, elliptical protrusions, polygonal protrusions, and irregular protrusions. In some examples, the protrusions are at least one of circular protrusions and regular polygonal protrusions. In order to facilitate the recognition of the signals of the marks 110, the shapes and sizes of the protrusions arranged on the surface where the first trackline 1101 and the second trackline 1102 are located are identical.

In some embodiments, the region where the tracklines (including the first trackline 1101 and the second trackline 1102) are located may also be provided as a groove, and the pores or protrusions may be provided in the groove, and the recognizable marks are provided in the pores or protrusions. For the arrangement of the pores or protrusions, and the instance where the recognizable marks are provided in the pores or protrusions, reference may be made to the above instance where "the first trackline 1101 and/or the second trackline 1102 includes pores or protrusions formed on the surface of the solid substrate 1, and at least part of the pores or protrusions are provided with a recognizable mark", which are not recited here.

According to the examples of the present application, the positions of the marking units 11 in the detection unit 10A can be determined by means of the signal distribution features and the positions of the marks 110 in the marking units 11 in the detection unit 10A. Furthermore, the recognition of the signals in the detection sites A10 in the detection unit 10A can be achieved by means of the positions of the marking units 11 in the detection unit 10A and the distribution features of the marking units 11.

According to the examples of the present application, the distribution features of the marking units 11 at least include the number and arrangement of the marks 110 constituting the marking units 11 and the positions of the marks 110, the intersecting manner of the first trackline 1101 and the second trackline 1102 in the mark 110 such as the included angle, and the length and width of the first trackline 1101 and the second trackline 1102 relative to the detection unit 10A.

In one possible implementation, the marks 110 in the marking unit 11 have a well-defined number and preset positions. The number of marks in each marking unit 11 is as described above, and will not be recited here for brevity.

The marks 110 in the marking unit 11 have a well-defined number and preset positions, which may be determined on the basis of the shape and size of the detection unit 10A. In one rectangular detection unit 10A corresponding to the shape and size of a FOV, three or more marks 110 are provided in the rectangular region block 10A1 formed by separately retracting the two pairs of opposite sides of the rectangular detection unit 10A by 2-4 folds. It will be appreciated that separately retracting the two pairs of opposite sides of the rectangular detection unit 10A by 2-4 folds does not mean that two pairs of opposite sides of the rectangular detection unit 10A are simultaneously retracted by the same folds, and illustratively, when one pair of opposite sides of the rectangular detection unit 10A are retracted by 2 folds, and the other pair of opposite sides are retracted by 3 folds, the rectangle defined by the extended lines of the two pairs of opposite sides is the rectangular region block 10A1.

Illustratively, the marking unit 11 includes four marks 110, the first tracklines 1101 and the second tracklines 1102 of the four marks 110 intersect perpendicularly, and the pattern formed by the connecting lines of the four intersections is a rectangle. At this time, the positions of the four marks 110 can achieve coarse positioning of the detection unit 10A, and divide the detection unit 10A into a plurality of regions; further, the precise positions of the signals produced in the detection sites A10 in each region is determined by the relative positions of the four intersections in the detection unit 10A and the lengths and the intersecting manner of the first trackline 1101 and the second trackline 1102.

In one possible implementation, the first trackline 1101 and the second trackline 1102 may intersect to form a symmetrical pattern, or may intersect in a non-symmetrical manner. In the examples of the present application, coarse positioning of the detection unit 10A may be achieved by setting the intersection manner of the first trackline 1101 and the second trackline 1102, and the detection unit 10A is divided into a plurality of regions.

Illustratively, as shown in FIG. 10, the marking unit 11 includes three marks 110, and the first tracklines 1101 and the second tracklines 1102 of the three marks 110 separately intersect perpendicularly. The three formed intersections are O1, 02, and 03, and the connecting lines of the three intersections form an isosceles triangle. In the marks 110 where the vertices are located, the first trackline 1101 and the second trackline 1102 intersect at their central points; of the two marks 110 located at the bottom corners, intersection O2 is close to the first end of the first trackline 1101 and the second end of the second trackline 1102 in the mark 110 where it is located, and intersection O3 is close to the second end of the first trackline 1101 and the first end of the second trackline 1102 in the mark 110 where it is located. As such, the positions of the three marks 110 can be determined by means of the signals of the marks 110 included in the marking unit 11, so as to achieve the region division in the detection unit 10A, i.e., to achieve the coarse positioning of the signals in the detection unit 10A; further, based on the intersection of the first trackline 1101 and the second trackline 1102 in each mark 110, signals for distinguishing the regions around each mark 110 are acquired, so as to achieve the positioning of the signal produced at the detection sites A10 in the detection unit 10A.

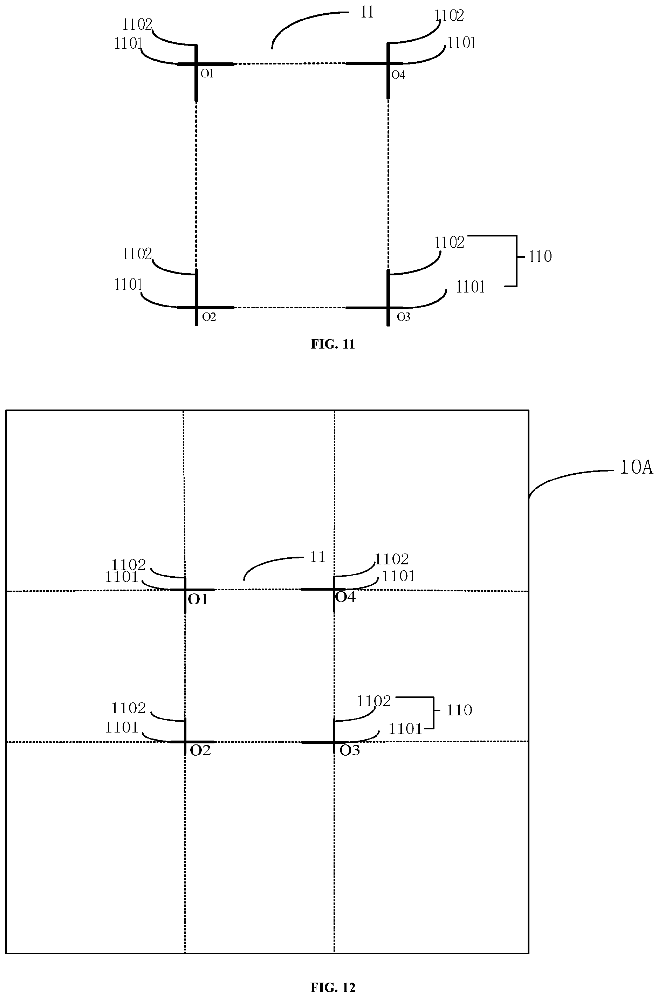

In another example, as shown in FIGs. 11 and 12, the marking unit 11 includes four marks 110, and the first tracklines 1101 and the second tracklines 1102 of the four marks 110 separately intersect perpendicularly. The four formed intersections are O1, 02, O3, and O4, and the connecting lines of the four intersections form a rectangle. The positions of the four marks 110 are determined by means of the signals of the marks 110 included in the marking unit 11, so as to achieve the region division in the detection unit 10A. For example, extended lines formed by the four sides of the rectangle divide the detection unit 10A into 3 × 3 region blocks, thus achieving the coarse positioning of the signals in the detection unit 10A. The four marks 110 intersect in different ways. For the mark 110 where intersection O1 is located, the intersection O1 is close to the first end of the first trackline 1101 and the first end of the second trackline 1102; for the mark 110 where intersection O2 is located, intersection O2 is close to the first end of the first trackline 1101 and the second end of the second trackline 1102; for the mark 110 where intersection O3 is located, intersection O3 is close to the second end of the first trackline 1101 and the second end of the second trackline 1102; for the mark 110 where intersection O4 is located, intersection O4 is close to the second end of the first trackline 1101 and the first end of the second trackline 1102. As such, on the basis of the 9 region blocks, the signals in each region block are acquired by means of the intersecting manner of the first trackline 1101 and the second trackline 1102 in each mark 110, so as to achieve the fine positioning of the signals generated at each detection site A10 in the detection unit 10A.

It will be appreciated that each trackline includes a first end and a second end that are opposite to each other, and when one end of the trackline is the first end, the other end of the trackline is the second end. In the illustrated examples, for the first trackline 1101, the end at the left is designated as the first end, and the end at the right is designated as the second end; for the second trackline 1102, the end upward is designated as the first end, and the end downward and rightward is designated as the second end.

According to the examples of the present application, the first trackline 1101 and the second trackline 1102 are tracklines having a certain aspect ratio. As one possible implementation, the first trackline 1101 has an aspect ratio of 15-30:1. Illustratively, the aspect ratio of the first trackline 1101 is 15:1, 18:1, 20:1, 22:1, 25:1, 28:1, 30:1, or the like. As one possible implementation, the second trackline 1102 has an aspect ratio of 15-30:1. Illustratively, the aspect ratio of the second trackline 1102 is 15:1, 18:1, 20:1, 22:1, 25:1, 28:1, 3 0:1, or the like. Firstly, by setting the aspect ratios of the first trackline 1101 and the second trackline 1102 within the above ranges, the difference between the region where the mark 110 is located and the surrounding detection regions can be enhanced, and the recognition intensity of the mark 110 can be improved; and secondly, by controlling the aspect ratios of the first trackline 1101 and the second trackline 1102 within the above range, the recognition intensity of the mark 110 can be increased within a limited area, and the areal proportion of the marking units 11 in the detection unit 10A can be reduced. In some examples, in one detection unit 10A, a 1% or less proportion of the area of the marking unit 11 to the area of the detection unit 10A still enables the recognition of the marking units 11. Illustratively, the area of the marking unit 11 may account for 1%, 0.9%, 0.8%, 0.7%, etc., of the area of the detection unit 10A. According to the examples of the present application, setting the intensities of the first tracklines 1101 and the second tracklines 1102 in the detection unit 10A may facilitate the recognition of the marks 110, and reduce the areal proportion of the marking units 11 in the detection unit 10A on this basis. In one possible embodiment, according to the distance *l* between centers of two adjacent detection sites A10 in the first direction, lengths and widths of the first trackline 1101 and the second trackline 1102 satisfy at least one of the following conditions:
the first trackline 1101 has a length of 80-150 *l*, for example, the length of the first trackline 1101 is 80 *l*, 85 *l,* 90 *l,* 95 *l*, 100 *l*, 105 *l*, 110 *l*, 115 *l*, 120 *l*, 125 *l*, 130 *l*, 135 *l*, 140 *l*, 145 *l*, 150 *l*, etc.;
the second trackline 1102 has a length of 80-150 *l*, for example, the length of the second trackline 1102 is 80 *l,* 85 *l,* 90 *l,* 95 *l*, 100 *l*, 105 *l*, 110 *l*, 115 *l*, 120 *l*, 125 *l*, 130 *l*, 135 *l*, 140 *l*, 145 *l*, 150 *l*, etc.; the first trackline 1101 has a width of 3-8 *l*, for example, the width of the first trackline 1101 is 3 *l,* 4 *l,* 5 *l,* 6 *1,* 7 *l,* 8 *l*, etc.;
the second trackline 1102 has a width of 3-8 *l*, for example, the width of the second trackline 1102 is 3 *l*, 4 *l,* 5 *l,* 6 *l,* 7 *l*, 8 *l*, etc.

In some examples, the first trackline 1101 has a length of 100-120 *l*; the second trackline 1102 has a length of 100-120 *l*; the first trackline 1101 has a width of 4-6 *l*; the second trackline 1102 has a width of 4-6 *l*. According to the examples of the present application, the distinction of the marking units may be enhanced by setting the length ratio of the first trackline 1101 to the second trackline 1102. In some examples, the length ratio of the first trackline 1101 to the second trackline 1102 is 0.8-1.2:1. Illustratively, the length ratio of the first and second tracklines 1101 and 1102 is 0.8:1, 0.9:1, 1:1, 1.1:1, 1.2:1, etc.

As such, the examples of the present application, by designing a plurality of sets of regularly arranged marking units 11 on the solid substrate, can achieve the coarse positioning of surface regions on the solid substrate 1 and the fine positioning of surface positions on the solid substrate 1 based on the arrangement rule of the marking units 11, the number and positions of marks 110, and the relation and features of the first tracklines 1101 and the second tracklines 1102. Moreover, the positioning on the surface of the solid substrate 1 by means of the arrangement of intersecting marking units can reduce the occupied area of the marks on the surface of the solid substrate 1 and the area loss on the surface of the solid substrate 1 and thus improve the valid utilization rate of the surface of the solid substrate 1. When the solid substrate 1 is used as a biochip, the sequencing throughput can be improved.

According to the examples of the present application, the recognizable marks are regularly distributed on the first trackline 1101 in the first arrangement rule, and the recognizable marks are regularly distributed on the second trackline 1102 in the second arrangement rule. It will be appreciated that the recognizable marks in the examples of the present application, whether provided in the grooves, the pores or protrusions, or even the processed surface pattern, may be formed by direct bonding, or may be formed by forming the detection site A10 at the corresponding position, placing the same sample in the detection site A10 as those in the detection sites A10 of the regions other than the marking units 11 in the detection unit 10A, and performing the same operation to achieve the introduction of the recognizable marks. In actual practice, for ease of operation, detection sites A10 same as those in the region other than the marking unit 11 are formed in the detection unit 10A, and subsequently the recognizable marks are introduced by the same operation. Hereinafter, for ease of understanding, the recognizable marks are illustrated by the introduction of detection sites A10 in mark 110. However, it will be appreciated that each detection site A10 in mark 110 corresponds to one recognizable mark.

In order to more clearly illustrate the arrangement of the marks 110, an example where the solid substrate 1 is a biochip for sequencing and the recognizable marks are optical marks such as fluorescent marks is described below.

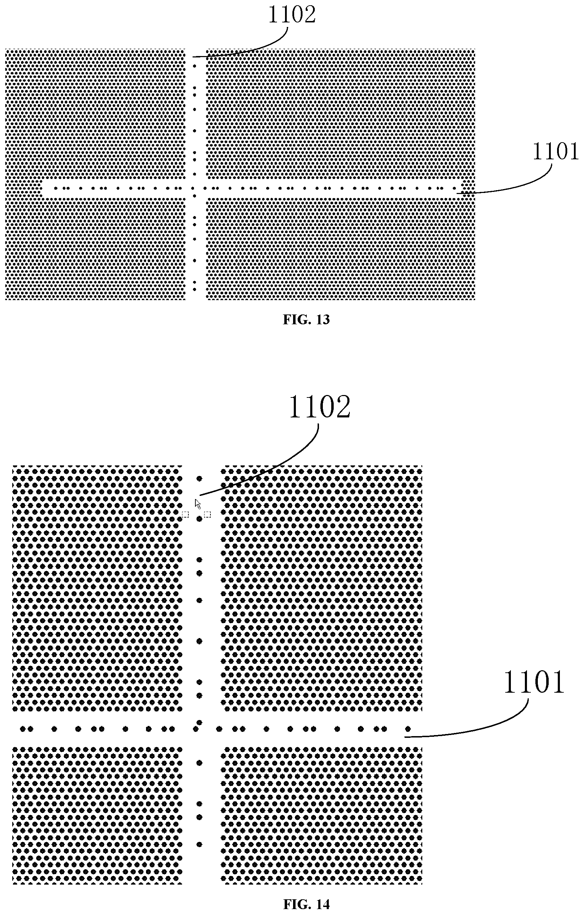

In one possible implementation, referring to FIGs. 13-16, the first trackline 1101 has first repeating units periodically arranged in the first direction. The first repeating unit includes a plurality of detection sites A10 arranged in the first direction, and the plurality of detection sites A10 on the first repeating unit are located on the same straight line, as shown in FIGs. 13 and 14. That is, all the detection sites A10 on the first trackline 1101 are located on the same straight line. As such, the recognizability of the detection sites A10 of the first trackline 1101 is improved, thus facilitating the recognition of the first trackline 1101. In some examples, the centers of all of the detection sites A10 on each of the first tracklines 1101 are located on the same straight line in the first direction. In this case, the centers of the optical signals produced at all the detection sites A10 on the first trackline 1101 are also generally on the same straight line, thus facilitating the recognition of the first trackline 1101.

The second trackline 1102 has second repeating units periodically arranged in the second direction. The second repeating unit includes a plurality of detection sites A10 arranged in the second direction, and the plurality of detection sites A10 on the second repeating unit are located on the same straight line. That is, all the detection sites A10 on the second trackline 1102 are located on the same straight line, as shown in FIGs. 13 and 14. As such, the recognizability of the detection sites A10 of the second trackline 1102 is improved, thus facilitating the recognition of the second trackline 1102. In some examples, the centers of all of the detection sites A10 on each of the second tracklines 1102 are located on the same straight line. In this case, the centers of the optical signals produced at all the detection sites A10 on the second trackline 1102 are also generally on the same straight line, thus facilitating the recognition of the second trackline 1102.

The periodically arranged repeating units allow the tracklines (including the first trackline 1101 and the second trackline 1102) in two directions to present a regular arrangement of spaced dark and bright optical signals, thus facilitating accurate recognition of the tracklines (including the first trackline 1101 and the second trackline 1102) in the two directions during optical detection, achieving the calibration of the detection units 10A, and improving the detection accuracy.

In one embodiment of the present application, the first repeating unit includes at least a set of consecutive sites. The consecutive sites include at least two first detection sites, and the distance between the two first detection sites is a first distance. Illustratively, the consecutive sites include two first detection sites. In some examples, the first repeating unit further includes a second detection site arranged on a first side of the consecutive sites. A second distance between a first detection site adjacent to the second detection site in the consecutive sites and the second detection site is greater than the first distance. As such, a blank region containing no detection sites is formed between the consecutive sites and the second detection site, and this region is present as a signal-free region such as a fluorescent dark region in an optical image acquired by the optical detection.

In some embodiments, the second distance ≥ the first distance × 2. In this case, the difference in brightness between the consecutive sites and the second detection site can be enhanced, contributing to the recognition of the first trackline 1101. Illustratively, the second distance = the first distance × 2.

In some examples, the first repeating unit further includes a second detection site arranged on a first side and a third site arranged on a second side of the consecutive sites. A second distance between a first detection site adjacent to the second detection site in the consecutive sites and the second detection site is greater than the first distance, and a third distance between a first detection site adjacent to the third detection site in the consecutive sites and the third detection site is greater than the first distance. It will be appreciated that the first side and the second side are based on the opposite sides of a reference object. For example, when the first repeating unit is located on the transverse axis, the first side of the consecutive sites refers to the left (or the right) of the consecutive sites, and on the contrary, the second side of the consecutive sites refers to the right (or the left) of the consecutive sites. Therefore, regions containing no detection sites are formed between the consecutive sites and the second detection site and between the consecutive sites and the third detection site, and the regions are present as dark regions in an optical image acquired by optical detection, such that a bright (second detection site)-dark-bright (consecutive sites)-dark-bright (third detection site) strip is formed, the arrangement of spaced dark and bright regions in the first direction presents higher regularity, and the recognizability of the first trackline 1101 is improved. In one example, when the third distance ≥ the first distance × 2, the difference in brightness between the consecutive sites and the third detection site can be enhanced, which is helpful for improving the recognition accuracy for the marking units 11. Illustratively, the third distance = the first distance × 3.

In the examples of the present disclosure, the second distance and the third distance may be identical or different. In one example, the second distance is different from the third distance. As such, the second detection site-consecutive site (first detection site)-third detection site arrangement is asymmetrical, which is beneficial to distinguishing the direction of the mark 110 and achieving the region recognition of the detection units 10A through the distinction.

In one embodiment, the first repeating unit includes a set of consecutive sites and a second detection site arranged on a first side of the consecutive sites, and the distance between the next detection site in the extension direction of the second detection site and the second detection site is greater than the first distance. As such, a region containing no detection sites is formed on both sides of the second detection site, such that a dark-light (second detection site)-dark-light (consecutive site) strip is present on the first side of the consecutive sites. The next detection site in the extension direction of the second detection site may be a detection site in the same first repeating unit where the second detection site is located, or may be a detection site in another adjacent first repeating unit. When the next detection site in the extension direction of the second detection site is a detection site in another adjacent first repeating unit, the first side of the consecutive sites in the first repeating unit is provided with the second detection site, and a dark region is arranged in the extension direction of the second detection site. As such, the first side of the first repeating unit contains no other detection sites. Illustratively, the distance between the next detection site in the extension direction of the second detection site and the second detection site = the first distance × 3. That is, a dark region with a length of the first distance is arranged in the extension direction of the second detection site.

In one example, the distance between the next detection site in the extension direction of the second detection site and the second detection site is greater than the first distance, and the distance between the next detection site in the extension direction of the third detection site and the third detection site is greater than the first distance. The next detection site in the extension direction of the second detection site may be a detection site in the same first repeating unit where the second detection site is located, or may be a detection site in another adjacent first repeating unit; similarly, the next detection site in the extension direction of the third detection site may be a detection site in the same first repeating unit where the third detection site is located, or may be a detection site in another adjacent first repeating unit. When the next detection site in the extension direction of the second detection site is a detection site in an adjacent first repeating unit, and the next detection site in the extension direction of the third detection site is a detection site in another adjacent first repeating unit, the first repeating unit consists of the second detection site, the consecutive sites, and the third detection site, and forms the arrangement of the second detection site, the consecutive sites, and the third detection site, and dark regions are arranged in the extension directions of the second detection site and the third detection site.

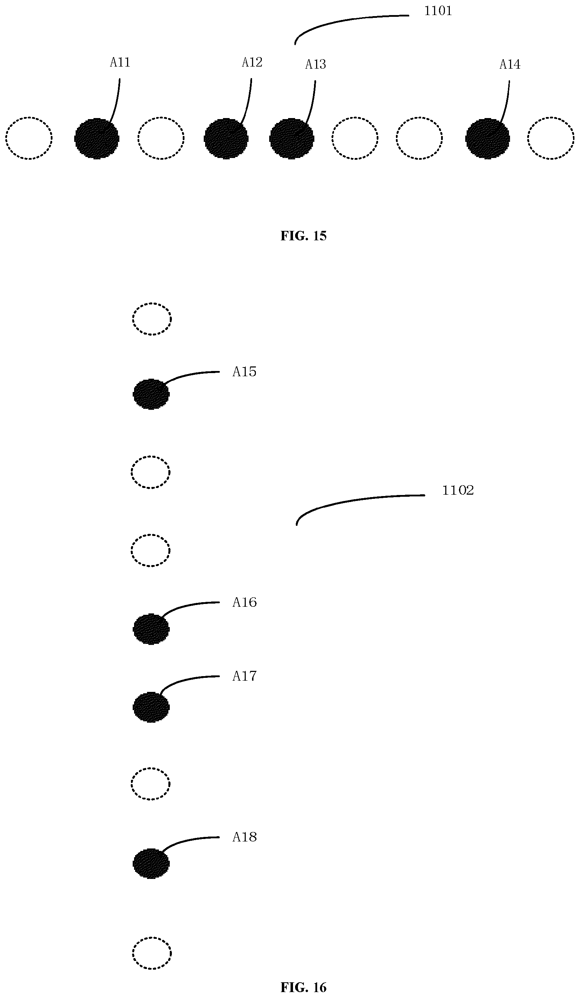

As shown in FIG. 15, in one first repeating unit of the first trackline 1101, open detection sites are indicated by solid circles, and blank sites are indicated by dashed circles and do not actually exist on the solid substrate. In this example, the blank sites are described for the purpose of illustrating the arrangement of detection sites. As such, in an optical image acquired by optically imaging the solid substrate, the regions corresponding to the solid circles are present as bright regions, and the regions between the solid circles, i.e., the regions where the dashed circles are located are present as dark regions in the optical image. The first repeating unit of the first trackline 1101 is provided with, from the first side to the second side, blank site-detection site A11-blank site-detection site A12-detection site A13-blank site-blank site-detection site A14 or detection site A 11-blank site-detection site A 12-detection site A 13-blank site-blank site-detection site A14-blank site in sequence, and in the first trackline 1101 formed by the detection sites and the blank sites, the distances between adjacent sites are all equal. It will be appreciated that the blanking site close to the first side and the blanking site close to the second side come from another adjacent first repeating unit. As such, one first repeating unit of the first trackline 1101 forms a dark-light-dark-long light (consecutive sites)-long dark (consecutive dark region)-light-dark stripe, such that the arrangement of spaced dark and bright regions in the first direction presents higher regularity, and the recognizability of the first trackline 1101 is improved.

In one embodiment of the present application, the second repeating unit includes at least a set of consecutive sites. The consecutive sites include at least two fourth detection sites, and the distance between the two fourth detection sites is a fourth distance. Illustratively, the consecutive sites include two fourth detection sites. In some examples, the second repeating unit further includes a fifth detection site arranged on a first side of the consecutive sites. A fifth distance between a fourth detection site adjacent to the fifth detection site in the consecutive sites and the fifth detection site is greater than the fourth distance. As such, a region containing no detection sites is formed between the consecutive sites and the fourth detection site, and this region is present as a signal-free region such as a fluorescent dark region in an image acquired by the optical detection.

In some embodiments, the fifth distance ≥ the fourth distance × 2. In this case, the difference in brightness between the consecutive sites and the fifth detection site can be enhanced, contributing to the recognition of the second trackline 1102 of the FOV 10. Illustratively, the fifth distance = the fourth distance × 3.

In some examples, the second repeating unit further includes a fifth detection site arranged on a first side and a sixth site arranged on a second side of the consecutive sites. A fifth distance between a fourth detection site adjacent to the fifth detection site in the consecutive sites and the fifth detection site is greater than the fourth distance, and a sixth distance between a fourth detection site adjacent to the sixth detection site in the consecutive sites and the sixth detection site is greater than the fourth distance. It will be appreciated that the first side and the second side are based on the opposite sides of a reference object. For example, when the second repeating unit is located on the longitudinal axis, the first side of the consecutive sites refers to the upper side of the consecutive sites, and on the contrary, the second side of the consecutive sites refers to the lower side of the consecutive sites. Therefore, regions containing no detection sites are formed between the consecutive sites and the fifth detection site and between the consecutive sites and the sixth detection site, and the regions are present as dark regions in an optical image acquired by optical detection, such that a bright (fourth detection site)-dark-bright (consecutive sites)-dark-bright (fifth detection site) strip is formed, the arrangement of spaced dark and bright regions in the second direction presents higher regularity, and the recognizability of the second trackline 1102 is improved. In one example, when the sixth distance ≥ the fourth distance × 2, the difference in brightness between the consecutive sites and the fifth detection site can be enhanced, which is helpful for improving the recognition accuracy for the marking units 11. Illustratively, the sixth distance = the fourth distance × 2.

In the examples of the present disclosure, the fifth distance and the sixth distance may be identical or different. In one example, the fifth distance is different from the sixth distance. As such, the fifth detection site-consecutive site (fourth detection site)-sixth detection site arrangement is asymmetrical, which is beneficial to distinguishing the direction of the mark 110 and achieving the region recognition of the detection units 10A through the distinction.

In one embodiment, the second repeating unit includes a set of consecutive sites and a fifth detection site arranged on a first side of the consecutive sites, and the distance between the next detection site in the extension direction of the fifth detection site and the fifth detection site is greater than the fourth distance. As such, a region containing no detection sites is formed on both sides of the fifth detection site, such that a dark-light (fifth detection site)-dark-light (consecutive site) strip is present on the first side of the consecutive sites. The next detection site in the extension direction of the fifth detection site may be a detection site in the same second repeating unit where the fifth detection site is located, or may be a detection site in another adjacent second repeating unit. When the next detection site in the extension direction of the fifth detection site is a detection site in another adjacent second repeating unit, the first side of the consecutive sites in the second repeating unit is provided with the fifth detection site, and a dark region is arranged in the extension direction of the fifth detection site. As such, the first side of the second repeating unit contains no other detection sites. Illustratively, the distance between the next detection site in the extension direction of the fifth detection site and the fifth detection site = the first distance × 3. That is, a dark region with a length of the first distance is arranged in the extension direction of the fourth detection site.

In one example, the distance between the next detection site in the extension direction of the fifth detection site and the fifth detection site is greater than the fourth distance, and the distance between the next detection site in the extension direction of the sixth detection site and the sixth detection site is greater than the fourth distance. The next detection site in the extension direction of the fifth detection site may be a detection site in the same second repeating unit where the fifth detection site is located, or may be a detection site in another adjacent second repeating unit; similarly, the next detection site in the extension direction of the sixth detection site may be a detection site in the same second repeating unit where the sixth detection site is located, or may be a detection site in another adjacent second repeating unit. When the next detection site in the extension direction of the fifth detection site is a detection site in an adjacent second repeating unit, and the next detection site in the extension direction of the sixth detection site is a detection site in another adjacent second repeating unit, the second repeating unit consists of the fifth detection site, the consecutive sites, and the sixth detection site, and forms the arrangement of the fifth detection site, the consecutive sites, and the sixth detection site, and dark regions are arranged in the extension directions of the fifth detection site and the sixth detection site. Illustratively, in adjacent second repeating units, the distance between the adjacent fifth detection site and sixth detection site = the fourth distance × 3. That is, a dark region with a length of the first distance is arranged in each of the extension directions of the fifth detection site and the sixth detection site.

As shown in FIG. 16, in one second repeating unit of the second trackline 1102, open detection sites are indicated by solid circles, and blank sites are indicated by dashed circles and do not actually exist on the solid substrate. In this example, the blank sites are described for the purpose of illustrating the arrangement of detection sites. As such, in an optical image acquired by optically imaging the solid substrate, the regions corresponding to the solid circles are present as bright regions, and the regions between the solid circles, i.e., the regions where the dashed circles are located are present as dark regions in the optical image. The second repeating unit of the second trackline 1102 is provided with, from the first side to the second side, blank site-detection site A15-blank site-blank site-detection site A16-detection site A17-blank site-detection site A18-blank site, or detection site A15-blank site-blank site-detection site A16-detection site A17-blank site-detection site A18-blank site-blank site, or blank site-blank site-detection site A15-blank site-blank site-detection site A16-detection site A17-blank site-detection site A18 in sequence, and in the second repeating unit formed by the detection sites and the blank sites, the distances between adjacent sites are all equal. It will be appreciated that the blanking site close to the first side and the blanking site close to the second side come from another adjacent first repeating unit. As such, one second repeating unit of the second trackline 1102 forms a dark-light-long dark (consecutive dark region)-long light (consecutive sites)-dark-light-dark stripe, such that the arrangement of spaced dark and bright regions in the second direction presents higher regularity, and the recognizability of the second trackline 1102 is improved.

According to the examples of the present application, the maximum diameter of the detection sites on the tracklines (including the first trackline 1101 and the second trackline 1102) may be identical to or different from the maximum diameter of the detection sites in the regions other than the tracklines in the detection unit. In one example, the maximum diameter of the detection sites on the tracklines (including the first trackline 1101 and the second trackline 1102) is greater than the maximum diameter of the detection sites in the regions other than the tracklines in the detection units, such that the tracklines (including the first trackline 1101 and the second trackline 1102) are more easily recognized. The maximum diameter is the linear distance between two points that are farthest apart from each other in the detection site. For a circular detection site, the diameter is the diameter of the circle.

In one example, the maximum diameter on the tracklines (including the first trackline 1101 and the second trackline 1102) is 400-500 nm, including but not limited to, 400 nm, 410 nm, 420 nm, 430 nm, 440 nm, 450 nm, 460 nm, 470 nm, 480 nm, 490 nm, 500 nm, etc. The maximum diameter of the detection sites on the first trackline 1101 may be identical to or different from the maximum diameter of the detection sites on the second trackline 1102.

In one example, the maximum diameter of the detection sites on the first trackline 1101 is identical to the maximum diameter of the detection sites on the second trackline 1102. Illustratively, the maximum diameter of all detection sites in the first and second directions is 450 nm.

**In a second aspect**, in one example, a biochip is provided, including the solid substrate according to any of the first aspect. This solid substrate is used for gene sequencing, and the detection regions on the solid substrate are sequencing regions, where fluorescence can be acquired at the pores. The calibration of FOVs according to the positions and settings of the marking units and marks in the marking units achieves the sequencing at the pore detection sites in the sequencing region and reduces the areal proportion of the marking units in the detection unit, thus facilitating the improvement of detection throughput.

The solid substrate may be a solid planar substrate made of silicon, glass wafer, or other material. Illustratively, the surface dimensions of the solid substrate are about 83 mm × 35 mm.

The sequencing method may be a sequencing-by-synthesis method. DNA polymerases, adapters, primers, and 4 types dNTPs with base-specific fluorescent labels are added simultaneously to the reaction system (such as the Sanger sequencing method). The 3'-OH of such dNTPs is chemically protected and only one dNTP is added at a time, which ensures that only one base is added at a time during the sequencing process. Also, after the dNTPs are added to the synthesized strand, all unused free dNTPs and DNA polymerase are discarded. Then, a buffer solution required for exciting fluorescence is added to excite fluorescence signals by laser. The fluorescence signals are recorded by an optical device, and finally, the optical signals are converted into sequencing bases by computer analysis. In the solid substrate of the present disclosure, the detection sites in the detection region are detection sites capable of emitting fluorescence signals after the excitation with laser, and the FOVs are pre-calibrated with the first tracklines 1101 and the second tracklines 1102, thus featuring high accuracy of sequencing. After the recording of the fluorescent signals, a chemical reagent is added to quench the fluorescent signals and the dNTP 3'-OH protecting groups are removed, such that the next cycle of sequencing can be conducted.

The present disclosure is suitable for any sequencing method based on optical signals, such as the second-generation sequencing, the third-generation sequencing, etc.

### Example 1

In this example, the biochip includes a plurality of rectangular detection units, and each detection unit includes a plurality of detection sites, and a set of marking units 11 as shown in FIGs. 11 and 12. Taking a detection unit 10A as an example, the marking unit 11 includes four marks 110, and each mark 110 includes a first trackline 1101 arranged in a first direction and a second trackline 1102 arranged in a second direction. The first trackline 1101 and the second trackline 1102 intersect perpendicularly, and the connecting lines of the four intersections form a rectangle. The lengths and widths of the first trackline 1101 and the second trackline 1102 are respectively identical. According to the distance *l* between centers of two adjacent detection sites in the first direction, the lengths and widths of the first trackline 1101 and the second trackline 1102 are as follows: the first trackline 1101 has a length of 100 *l* and a width of 5 *l*; the second trackline 1102 has a length of 100 *l* and a width of 5 *l*. Circular pores with a diameter of 300 nm are provided in the first trackline 1101, and the size of the circular pores is identical to that of the detection sites in the detection unit 10A. The first repeating unit constituting the first trackline 1101 has a structure shown in FIG. 15, and is provided with, from the first side to the second side, blank site-detection site A11-blank site-detection site A12-detection site A13-blank site-blank site-detection site A14 or detection site A 11-blank site-detection site A 12-detection site A 13-blank site-blank site-detection site A 14-blank site in sequence, and in the first trackline 1101 formed by the detection sites and the blank sites, the distances between adjacent sites are all equal. The second repeating unit constituting the second trackline 1102 has a structure shown in FIG. 16, and is provided with, from the first side to the second side, blank site-detection site A 15-blank site-blank site-detection site A 16-detection site A 17-blank site-detection site A 18-blank site, or detection site A15-blank site-blank site-detection site A16-detection site A17-blank site-detection site A18-blank site-blank site, or blank site-blank site-detection site A15-blank site-blank site-detection site A 16-detection site A 17-blank site-detection site A18 in sequence, and in the second repeating unit formed by the detection sites and the blank sites, the distances between adjacent sites are all equal.

### Example 2

In this example, the biochip has a size identical to that of Example 1 and includes a plurality of rectangular detection units identical to those of Example 1, and each detection unit includes a plurality of detection sites, and a set of marking units 11 as shown in FIGs. 11 and 12. Taking a detection unit 10A as an example, the marking unit 11 includes four marks 110, and each mark 110 includes a first trackline 1101 arranged in a first direction and a second trackline 1102 arranged in a second direction. The first trackline 1101 and the second trackline 1102 intersect perpendicularly, and the connecting lines of the four intersections form a rectangle. The lengths and widths of the first trackline 1101 and the second trackline 1102 are respectively identical. According to the distance *l* between centers of two adjacent detection sites in the first direction, the lengths and widths of the first trackline 1101 and the second trackline 1102 are as follows: the first trackline 1101 has a length of 100 *l* and a width of 5 *l*; the second trackline 1102 has a length of 100 *l* and a width of 5 *l*. As shown in FIG. 9, the first trackline 1101 and the second trackline 1102 are grooves formed on the surface of the solid substrate, and each groove has a continuous region to which a recognizable mark can be bound. For example, the groove is a surface-processed groove to which a nucleic acid sample having a density 10 folds or greater than the density of detection sites in the sequencing unit 1 is bound. During the sequencing, the nucleic acid sample may bind to a nucleic acid substrate with a recognizable mark, such that the first trackline 1101 and the second trackline 1102 in the mark 110 present a continuous, high-intensity signal.

### Comparative Example

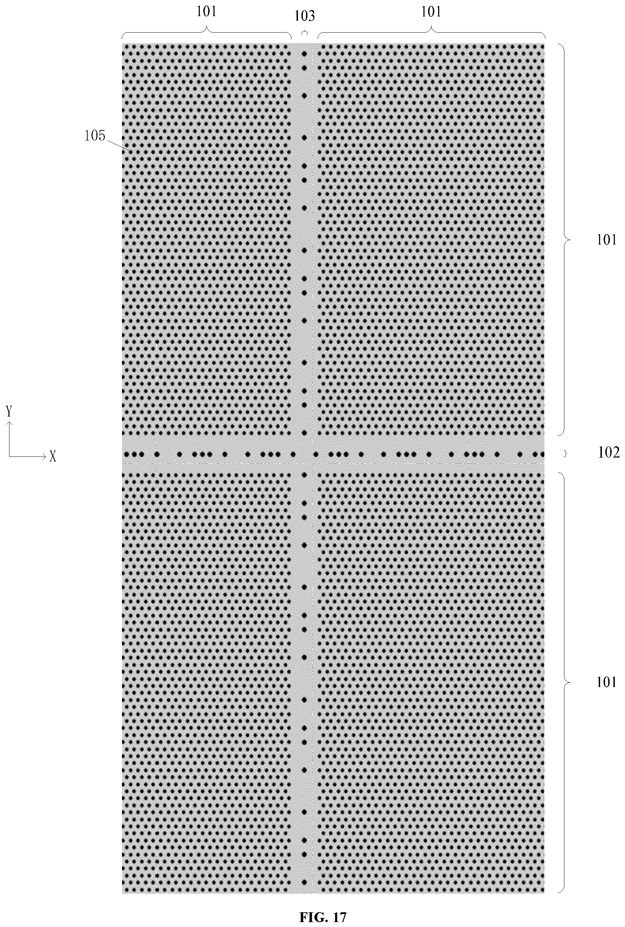

In this comparative example, the biochip has a size identical to that of Example 1 and includes a plurality of rectangular detection units identical to those of Example 1 (except for the marks, the other regions in the rectangular detection units have detection sites with the same arrangement, size, and shape). As shown in FIGs. 1-3 and 17, each detection unit is provided with tracklines. The tracklines include a first trackline 102 provided in the first direction (or X direction) and a second trackline 103 provided in the second direction (or Y direction). The first trackline 102 and the second trackline 103 intersect perpendicularly, and the width of the tracklines is identical to that of the tracklines of Examples 1 and 2. The first trackline 102 and the second trackline 103 divide the rectangular detection unit into a plurality of detection regions 101. The tracklines are provided with circular pore structures. The aperture of the sites on the first trackline 102 is 450 nm, and the aperture of the sites on the second trackline 103 is 450 nm.

In the sample detection region 101, the detection sites 105 are configured as circular pore structures with an aperture of 300 nm. The distance between adjacent detection sites in the X direction is 750 nm, and the distance between adjacent detection sites in the Y direction is 1300 nm. The distance between adjacent sites on the first trackline 102 is 750 nm, and the distance between adjacent sites on the second trackline 103 is 1300 nm. The perpendicular distance between the boundary of the first trackline 102 and the boundary of the adjacent detection region 101 is 1500 nm, and the perpendicular distance h between the boundary of the second trackline 103 and the boundary of the adjacent detection region 101 is 2100 nm. The aperture of the sites on the first trackline 102 is 450 nm, and the aperture of the sites on the second trackline 103 is 450 nm.

The chips provided in Examples 1 and 2 and Comparative Example were subjected to sequencing by synthesis in the same conditions, and Q30, mapped rate, error, output reads, and the like of the sequencing were summarized.

The Q30 means the ratio that the accuracy rate of sequenced bases is 99.9%; for example, a Q30 ≥ 85 means that the ratio of the sequenced bases with an accuracy rate of 99.9% is greater than or equal to 85%;

The mapped rate denotes the match rate, and refers to the percentage of matching nucleic acid fragments when aligned to a sample nucleic acid sequence;

The error denotes the error rate of sequencing;

The output reads denote the amount of reads data output per unit area, where a greater number of reads output per unit area denotes a higher surface sequencing throughput.

Data of the biochip of Example 1 are shown in Table 1 below:

**Table 1**

| | Number | Mapped rate | Error | Q30 | Output Reads |
|---|---|---|---|---|---|
| Example 1 | V03 | 86.99% | 0.36% | 86.8% | 2472M |
| Example 2 | V04 | 89.78% | 0.27% | 89.3% | 2761M |
| Comparative Example | V02 | 89.54% | 0.28% | 89.1% | 2753M |

As can be seen from the data in Tables 1, compared with the trackline markings in the Comparative Example, among the key sequencing data of the biochips in Examples 1 and 2, the mapped rate was improved, the error was reduced, and the Q30 was improved, suggesting that when the marked biochips in Examples 1 and 2 of the present application are used for sequencing, the tracklines (including the first trackline 1101 and the second trackline 1102) have better recognizability, thus facilitating calculations via an algorithm and effectively improving the sequencing accuracy; also, the output reads corresponding to Examples 1 and 2 were higher than that of Comparative Example 1, suggesting that when the marked biochips in Examples 1 and 2 of the present application are used for sequencing, the sequencing throughput can be improved, which may be attributed to the reduction of the areal proportion of the marking units in the detection unit provided by the examples of the present application.

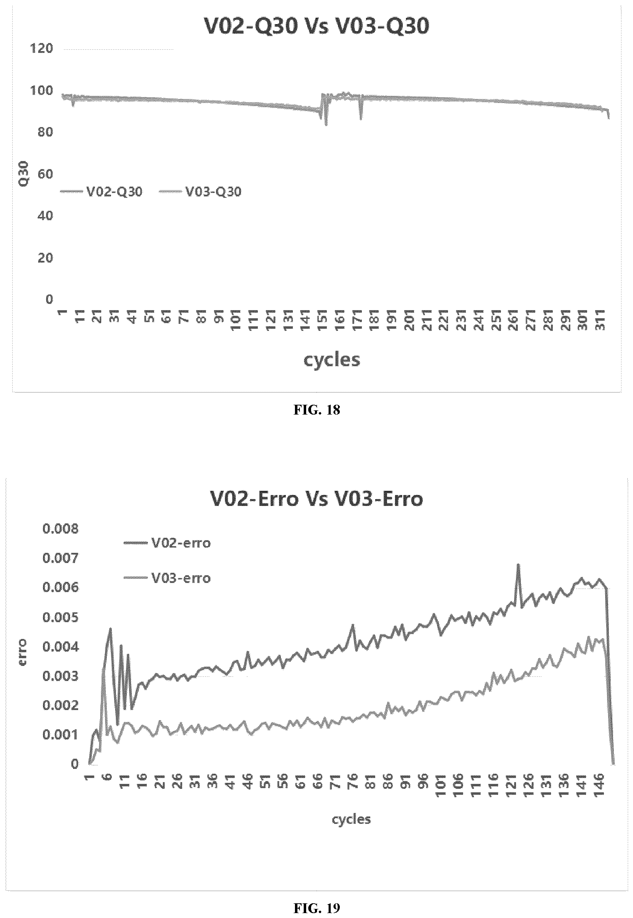

FIG. 18 illustrates Q30 curves for Example 1 (V03) and Comparative Example 1 (V02). As can be seen, both Example 1 (V03) and Comparative Example (V02) exhibited high Q30, and Q30 of Example 1 (V03) was higher than that of Comparative Example.

FIG. 19 illustrates error rate curves for Example 1 (V03) and Comparative Example 1 (V02). As can be seen, Example 1 (V03) exhibited a lower error rate than that of Comparative Example (V02).

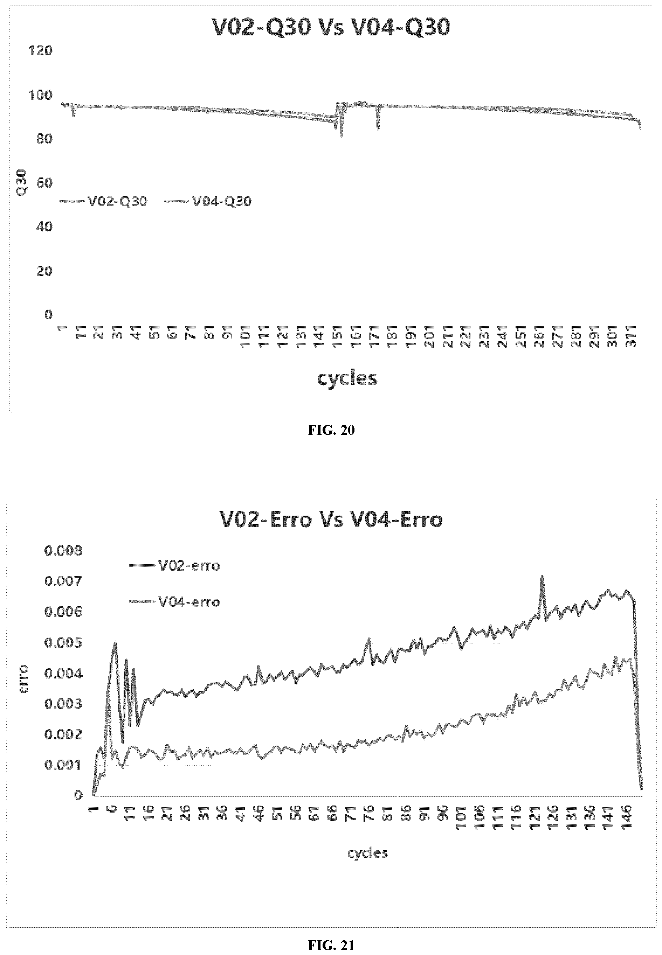

FIG. 20 illustrates Q30 curves for Example 2 (V04) and Comparative Example 1 (V02). As can be seen, both Example 2 (V04) and Comparative Example (V02) exhibited high Q30, and Q30 of Example 2 (V04) was higher than that of Comparative Example.

FIG. 21 illustrates error rate curves for Example 2 (V04) and Comparative Example 1 (V02). As can be seen, Example 2 (V04) exhibited a lower error rate than that of Comparative Example (V02).

The present disclosure has been illustrated by means of specific examples, which, however, are provided to help understand the present disclosure, rather than limiting the present disclosure. Numerous simple deductions, modifications, or substitutions may also be made by those skilled in the art in light of the present teachings.

## Claims

1. A solid substrate, wherein a surface of the solid substrate is provided with a plurality of sets of regularly arranged marking units; each set of marking units comprises at least three marks arranged according to a preset pattern; each mark comprises a first trackline arranged in a first direction and a second trackline arranged in a second direction and intersected with the first trackline.

2. The solid substrate according to claim 1, wherein the solid substrate is provided with at least one detection region, and each detection region is provided with at least one set of marking units.

3. The solid substrate according to claim 2, wherein the detection region comprises one or more detection units, and each detection unit is provided with one set of marking units.

4. The solid substrate according to claim 3, wherein the detection region comprises a plurality of detection rows arranged in the first direction;
optionally, the distances between adjacent detection rows are identical;
optionally, a plurality of uniformly spaced detection sites are arranged in the same row;
optionally, the detection sites are micron- or nano-pores, or the detection sites are nano-scale or micron-scale protrusions;
optionally, the detection sites in the detection region have the same size;
optionally, in two adjacent rows, except for at least one of the two detection sites at the two ends of each row, each detection site on one row is located on the perpendicular bisector of the line segment between two adjacent detection sites on the other row.

5. The solid substrate according to claim 3 or 4, wherein the detection unit comprises a rectangular region block located in a central region of the detection unit;
optionally, the marking unit is arranged in the rectangular region block; and
in one set of marking units, an area of the region defined by the connecting lines of the marks preferably accounts for 50% or more of the total area of the rectangular region block.

6. The solid substrate according to any one of claims 1-5, wherein in one set of marking units, the preset pattern defined by the connecting lines of the marks is a symmetrical pattern;
optionally, the center of the symmetric pattern coincides with the center of the detection unit;

7. The solid substrate according claim 6, wherein:
each set of marking units comprises three marks arranged according to the preset pattern, optionally, the pattern defined by the connecting lines of the three marks is an isosceles triangle; or
each set of marking units comprises four marks arranged according to the preset pattern,
optionally, the pattern defined by the connecting lines of the four marks is a rectangle;
optionally, when the detection unit comprises the rectangular region block located in the central region of the detection unit, sides of the rectangular region block are parallel or perpendicular to the first direction; and
the four marks are respectively arranged at four vertices of the rectangular region block.

8. The solid substrate according to any one of claims 1-7, wherein the first direction and the second direction are perpendicular;
and/or, the area of the marking units accounts for 1 % or less of the area of the detection unit.

9. The solid substrate according to any one of claims 1-8, wherein the first trackline has an aspect ratio of 15-30: 1; and/or
the second trackline has an aspect ratio of 15-30: 1; and/or the length ratio of the first trackline to the second trackline is 0.8-1.2:1;

10. The solid substrate according to claim 9, wherein the solid substrate comprises a plurality of detection units, and each detection unit is provided with one set of marking units; the detection unit is provided with a plurality of uniformly spaced detection sites arranged in the first direction;
a distance between centers of two adjacent detection sites in the first direction is *l*, lengths and widths of the first trackline and the second trackline satisfy at least one of the following conditions:
the first trackline has a length of 80-150 *l*,
the second trackline has a length of 80-150 *l*,
the first trackline has a width of 3-8 *l*,
the second trackline has a width of 3-8 *l*.

11. The solid substrate according to any one of claims 1- 10, wherein the first trackline and/or the second trackline are grooves formed on the surface, and a recognizable mark is arranged in the groove;

12. The solid substrate according to claim 11, wherein when the solid substrate comprises a plurality of detection units, each detection unit is provided with one set of marking units, and the detection unit is provided with a plurality of uniformly spaced detection sites arranged in the first direction;
a distance between two adjacent recognizable marks in the groove is less than the distance between two adjacent detection sites in the detection unit;
the distance between the recognizable marks in the groove is preferably 1/2 or less of the distance between adjacent detection sites in the detection unit.

13. The solid substrate according to any one of claims 1-12, wherein the first trackline and/or the second trackline comprises pores or protrusions formed on the surface of the solid substrate, and at least part of the pores or protrusions are provided with a recognizable mark;
optionally, in the first trackline, the center-connecting lines of the pores or protrusions form a straight line which is parallel to the first direction; and/or in the second trackline, the center-connecting lines of the pores or protrusions form a straight line which is perpendicular to the first direction.

14. The solid substrate according to claim 13, wherein
distances between the adjacent recognizable marks on the first trackline and/or the second trackline are separately equal; or
when the solid substrate comprises a plurality of detection units, each detection unit is provided with one set of marking units; and the detection unit is provided with a plurality of uniformly spaced detection sites arranged in the first direction; the distance between at least part of adjacent recognizable marks is greater than the distance between two adjacent detection sites;
preferably, the distance between centers of two adjacent detection sites in the first direction is *l*, the distance between adjacent recognizable marks is n folds of *l*, and n is an integer greater than or equal to 1 or n is a positive integer of 2 - 4.

15. A biochip, comprising the solid substrate according to any one of claims 1-14.
